# EUROPÄISCHE PATENTSCHRIFT

(11) **EP 2 895 421 B1**
(45) Veröffentlichungstag und Bekanntmachung des Hinweises auf die Patenterteilung: **01.01.2020**
(21) Anmeldenummer: 13753107.5
(22) Anmeldetag: 24.08.2013
(51) Int. Cl.: B67B 3/00

(54) **VORRICHTUNG UND VERFAHREN ZUM STERILISIEREN VON VERSCHLÜSSEN FÜR BEHÄLTER**
DEVICE AND METHOD FOR THE STERILIZATION OF CLOSURES FOR CONTAINERS
DISPOSITIF ET PROCÉDÉ DE STÉRILISATION DE BOUCHONS DE RÉCIPIENTS

(30) Priorität: 11.09.2012 DE 102012017986
(43) Veröffentlichungstag der Anmeldung: 22.07.2015
(73) Patentinhaber: KHS GmbH, 44143 Dortmund (DE)
(72) Erfinder: NIEHR, Thomas, 55583 Bad Münster am Stein Ebernburg (DE); VORWERK, Jürgen, 56290 Mörsdorf (DE); SINGUR, Igor, 55545 Bad Kreuznach (DE)
(86) Internationale Anmeldenummer: PCT/EP2013/002554
(87) Internationale Veröffentlichungsnummer: WO 2014/040694

(56) Entgegenhaltungen:
- EP-A2- 1 801 066
- WO-A2-2010/031464
- DE-A1-102010 025 541
- DE-A1-102010 052 207

## Beschreibung

Die Erfindung bezieht sich auf eine Vorrichtung zum Entkeimen bzw. Sterilisieren von Verschlüssen gemäß dem Oberbegriff des Patentanspruchs 1, sowie auf ein Verfahren, das die besagte Vorrichtung verwendet.

Beispielsweise bei Anlagen der Getränkeindustrie ist es üblich und bekannt, die zum Verschließen von gefüllten Flaschen oder anderen Behältern verwendeten Verschlüsse, insbesondere auch kappenartige Verschlüsse, wie Schraubverschlüsse, Flachkappen, Kronkorken usw. vor ihrer Verwendung, d.h. vor dem Aufbringen auf den jeweiligen Behälter zu sterilisieren bzw. zu entkeimen, und zwar u.a. unter Verwendung von Sterilisiermitteln, die aus H₂O₂-Dampf (verdampfte wässrige Wasserstoffperoxid-Lösung mit ausreichend hoher H2O2-Konzentation) oder aus einem mit H₂O₂-Aerosol angereicherten gas- und/oder dampfförmigen Trägermedium bestehen. Um eine wirksame Sterilisation mit hoher Entkeimungsrate zu erreichen, werden die Verschlüsse vor der Beaufschlagung mit dem Sterilisiermittel vorzugsweise vorgewärmt, beispielsweise auf eine Temperatur im Bereich zwischen 50°C und 85°C und dann nach der Behandlung bzw. Beaufschlagung mit dem gas- und/oder dampfförmigen Sterilisiermittel unter Verwendung eines vorzugsweise erwärmten sterilen gas- und/oder dampfförmigen Mediums, beispielsweise unter Verwendung von vorzugsweise erwärmter steriler Luft getrocknet, sodass die sterilisierten Verschlüsse dann anschließend zum Verschließen von Behältern verwendet bzw. hierfür einer Verschließmaschine zugeführt werden können.

Eine bekannte Vorrichtung zum Sterilisieren von Verschlüssen für Behälter mit H₂O₂-Aerosol oder Dampf weist zwei um eine gemeinsame vertikale Maschinenachse umlaufend angetriebene Rotoren auf (DE 10 2010 025 541 A1, die eine gattungsgemäße Vorrichtung offenbart).

Jeder Rotor ist in einem Behandlungsraum aufgenommen. An jedem Rotor ist um die Maschinenachse versetzt eine Vielzahl von Verschlussaufnahmen vorgesehen, die jeweils zur Aufnahme einer Vielzahl von Verschlüssen in Reihe ausgebildet sind und sich in einer Achsrichtung parallel zur Maschinenachse erstrecken. Mit den Verschlussaufnahmen werden die Verschlüsse bei umlaufenden Rotoren durch die Behandlungsräume zwischen einer Verschlussaufgabe und einer Verschlussabgabe bewegt. In den Behandlungsräumen erfolgt jeweils eine Beaufschlagung der Verschlüsse mit Heißluft, und zwar im Behandlungsraum des oberen Rotors für ein Vorerwärmen der Verschlüsse und in dem Behandlungsraum des unteren Rotors für ein Aktivieren des Sterilisationsmediums (H₂O₂-Aerosol) und für ein Trocknen der Verschlüsse. Die Beaufschlagung der Verschlüsse mit dem Sterilisationsmedium erfolgt auf einer parallel zur Maschinenachse orientierten Behandlungsstrecke, die die Verschlussabgabe des oberen Rotors mit der Verschlussaufgabe des unteren Rotors verbindet und durch die die Verschlüsse durch Schwerkraft hindurchbewegt werden.

Nachteilig hierbei ist neben der durch die zwei Rotoren bedingten erheblichen Baugröße insbesondere, dass die Behandlungszeit für die Beaufschlagung der Verschlüsse mit dem Sterilisiermittel und auch die Taktzeit der getakteten Bewegung der Rotoren von der Fallgeschwindigkeit abhängen, mit der sich die Verschlüsse durch die Behandlungsstecke bewegen. Weiterhin ist bei vorgenannter Sterilisationseinheiten problematisch, dass durch die Aufwärmung die Verschlüsse an Formstabilität verlieren, weil diese weiche werden, was nachfolgend beim Transport und der freien Bewegung untereinander zu Maschinenstörungen führen kann.

In der DE102010052207A1 wurde weiterhin eine Vorrichtung vorgeschlagen, , die zum Sterilisieren von Verschlüssen durch Beaufschlagung mit UV-Strahlung dient und bei der die Verschlüsse in vertikal ausgerichteten, käfigartigen Verschlussaufnahmen aufgenommen sind, die am Umfang eines um eine vertikale Maschinenachse umlaufend antreibbaren Rotors gebildet und mit ihrer Längserstreckung parallel zur Rotorachse orientiert sind. Die Verschlüsse werden dabei mit dem Rotor bzw. mit den Verschlussaufnahmen auf einer Behandlungsstrecke zwischen einer Verschlussaufgabe und einer Verschlussabgabe einer Vielzahl von mit dem Rotor nicht umlaufenden UV-Strahlern vorbei bewegt.

Aufgabe der Erfindung ist es, eine Vorrichtung aufzuzeigen, die eine hohe Qualität der Sterilisation und/oder Entkeimungsrate ermöglicht, wobei eine gezielte Sterilisation der einzelnen Verschlüsse mit hohem, störungsfreiem Durchsatz sichergestellt ist.

Die Aufgabe wird ausgehend vom Oberbegriff des unabhängigen Patentanspruch 1 durch dessen kennzeichnende Merkmale gelöst.

Der wesentliche Aspekt der erfindungsgemäßen Vorrichtung zum Sterilisieren von Verschlüssen zum Verschließen von Flaschen oder dergleichen Behältern, insbesondere zum Sterilisieren von kappenartigen Verschlüssen, mit einem Transportsystem zum Bewegen der Verschlüsse durch mehrere Behandlungszonen, in der die Verschlüsse sterilisiert, sowie aktiviert und/oder getrocknet werden. Dabei weist das Transportsystem in der wenigstens einen Behandlungszone wenigstens einen um eine Maschinen- oder Rotorachse umlaufend antreibbaren Rotor mit einer Vielzahl von am Umfang dieses Rotors ausgebildeten Verschlussaufnahmen auf. Mittels dieser Verschlussaufnahmen werden die Verschlüsse durch mehrere Behandlungszonen auf einer Behandlungsstrecke zwischen einer Verschlussaufgabe und einer Verschlussabgabe bewegt. Dabei sind an der wenigstens einen Behandlungsstrecke eine oder mehrere Applikationsstationen vorgesehen, mit einer auf zumindest eine Verschlussaufnahme gerichteten Sterilisiermittelsprühvorrichtung zum gesteuerten und/oder gezielten Beaufschlagen der einzelnen in der Verschlussaufnahme aufgenommenen und gehaltenen Verschlüsse mit Wasserstoffperoxid-Aerosol/-Dampf, einem Wasserstoffperoxid-Aerosol enthaltenden Sterilisiermittel und/oder einem Gas zum Aktivieren des Wasserstoffperoxids und Trocknen der Oberflächen..

Besondere Vorteile bestehen u.a. darin, dass Gruppen von Verschlüssen ruhend in den Verschlussaufnahmen gehalten werden, d.h. die Verschlüsse in den Verschlussaufnahmen bewegen sich weder relativ zueinander noch zur Verschlussaufnahme selbst, während der gesamten Behandlungszeit. Durch diese einfache Führung der Verschlüsse während der Behandlung, ist ein absolut störungsfreier Fluss dieser Verschlüsse ermöglicht, so dass hohe Leistung trotz der sehr kompakten konstruktiven Bauform und des geringen Bauvolumens erreicht *wird. Insbesondere werden gleichbleibende, d.h. reproduzierbar hohe Entkeimungsraten bei der Sterilisation erreicht.

Nachfolgend sollen unter dem Begriff "Verschluß" Verschlüsse unterschiedlichster Art verstanden werden, insbesondere kappenartige Verschlüsse, wie Schraubverschlüsse, Flachkappen, Kronkorken usw.

Unter dem Begriff "Wasserstoffperoxid-Aerosol oder H₂O₂-Aerosol" soll nachfolgend ein Gemisch verstanden werden, welches mindestens ein Trägergas, insbesondere Luft, und weiterhin H₂O₂ oder eine H₂O₂-haltige Flüssigkeit, insb. eine wässrige Lösung enthält. Dabei kann das H₂O₂ oder die H₂O₂-Lösung atomisiert in das Trägergas eingebracht worden sein bzw. vorliegen (bspw. mittels einer Düse) und/oder durch Energieeintrag verdampft und darin gemischt sein. Bevorzugt wird ein heißes Wasserstoffperoxid-Aerosol eingesetzt.

In einem bevorzugten Ausführungsbeispiel sind an der zumindest einen Applikationsstation zumindest zwei einander gegenüberliegende Sterilisiermittelsprühvorrichtungen vorgesehen, die zur gezielten Beaufschlagung der einzelnen in einer Verschlussaufnahme aufgenommenen Verschlüsse mit Wasserstoffperoxid-Aerosol von gegenüberliegenden Seiten ausgebildet sind. Dadurch wird eine möglichst vollständige Beaufschlagung der Oberfläche der Verschlüsse mit Sterilisiermittel und damit eine optimale Sterilisierwirkung erreicht.

In einem bevorzugten Ausführungsbeispiel sind in Umlaufrichtung des Rotors aufeinander folgend mehrere Applikationsstationen zum gezielten Beaufschlagen der in den Verschlussaufnahmen aufgenommenen Verschlüsse mit Wasserstoffperoxid-Aerosol vorgesehen. Dadurch kann der Aufbringvorgang in mehreren zeitlich voneinander getrennten Schritten durchgeführt werden, wodurch sich eine weitere Steigerung der Sterilisierqualität ergibt.

Bevorzugt sind die Verschlussaufnahmen in festen Winkel- oder Teilungsabständen um die Maschinen- oder Rotorachse verteilt angeordnet ist und die aufeinanderfolgenden Applikationsstationen sind zueinander um mindestens zwei Winkel- oder Teilungsabstände zueinander beabstandet. Bei einer getakteten, d.h. intermittierenden Drehbewegung des Rotors um jeweils einen Winkel- oder Teilungsabstand pro Takt ergibt sich nach der Behandlung der in einer Verschlussaufnahme enthaltenen Verschlüsse in der ersten Applikationsstation eine Verweilzeit der Verschlüsse an einer zwischen den Applikationsstationen angeordneten Einwirkstation von mindestens einer Taktzeit. Während dieser Zeit kann das Sterilisiermittel auf die Verschlüsse einwirken. Anschließend werden diese Verschlüsse nach einer weiteren getakteten Drehbewegung des Rotors der zweiten Applikationsstation zugeführt, in der eine weitere Beaufschlagung der Verschlüsse mit Sterilisiermittel erfolgt. Durch die Beabstandung der Applikationsstationen zueinander wird wiederum eine Steigerung der Sterilisierqualität erreicht.

In einem weiteren bevorzugten Ausführungsbeispiel weist die Sterilisiermittelsprühvorrichtung jeweils eine Vielzahl von Auslässen auf, wobei die aufeinanderfolgenden Auslässe jeweils um einen Verschlussdurchmesser oder im Wesentlichen einen Verschlussdurchmesser in Richtung der Längserstreckung der Verschlussaufnahmen beabstandet sind. Weiterhin bevorzugt sind die Auslässe derart angeordnet, dass jeweils die Auslässe mittig, d.h. achsgleich, oder im Wesentlichen mittig (achsgleich) in Bezug auf die in den Verschlussaufnahmen vorgesehenen Verschlüsse zu liegen kommen. Damit ist sichergestellt, dass jeweils einem Verschluss ein Auslass zugeordnet ist und damit dieser Verschluss einzeln und gezielt mit dem Sterilisiermittel beaufschlagt wird.

Bevorzugt sind die einzelnen Auslässe der Sterilisiermittelsprühvorrichtung und/oder die Sterilisiermittelsprühvorrichtungen unterschiedlicher Applikationsstationen mit einer zentralen Sterilisiermittelzuführung verbunden. Dadurch ist eine zentrale Speisung sämtlicher Auslässe der Sterilisiermittelsprühvorrichtungen mittels einer einzigen Sterilisiermittelzuführung möglich.

In Umlaufrichtung auf die zumindest eine Applikationsstation folgend ist zumindest eine Aktivierungs- und/oder Trocknungsstation vorgesehen. An dieser Aktivierungs- und Trocknungsstation werden die Verschlüsse mit einem Trocknungs- und Aktivierungsmedium, vorzugsweise unter Verwendung von erwärmter, steriler Luft getrocknet, wobei die Trocknung weiterhin vorzugsweise jeweils gezielt durch den jeweiligen Verschlüssen zugeordnete Düsen bzw. Auslässe erfolgt, an denen die Luft gerichtet auf die einzelnen Verschlüsse abgegeben wird.

Dieses Trocknen der Verschlüsse bewirkt insbesondere zu Beginn der Beaufschlagung des Verschlusses mit dem Trocknungs- und Aktivierungsmedium ein Aktivieren des im Sterilisiermedium enthaltenen Wasserstoffperoxids (H₂O₂), so dass dieses in seine Zerfallsprodukt zerfällt und die Sterilisierung bewirkt.

In einem bevorzugten Ausführungsbeispiel ist in Umlaufrichtung auf die zumindest eine Applikationsstation folgend vor der zumindest einen Aktivierungs- und Trocknungsstation zumindest eine Einwirkstation vorgesehen. Diese Einwirkstation dient der Gewinnung einer Zeitspanne zwischen dem Aufbringen des Sterilisiermediums und Trocknung bzw. Aktivierung, so dass das Sterilisiermittel über einen gewissen Zeitraum an den Verschlüssen wirken kann.

Besonders bevorzugt ist der wenigstens eine Rotor hohltrommel- oder hohlzylinderartig mit einem von einer Vielzahl von Verschlussaufnahmen gebildeten käfigartigen Trommelmantel ausgeführt, wobei die Verschlussaufnahmen ihrerseits bevorzugt jeweils käfigartig mit sich in Längsrichtung der jeweiligen Verschlussaufnahme erstreckenden stab- und/oder stangenförmigen Verschlussführungsschienen ausgebildet sind, zwischen denen die Verschlüsse in der jeweiligen Verschlussaufnahme aufgenommen sind. Dadurch wird erreicht, dass die sich in den Verschlussaufnahmen befindlichen Verschlüsse nahezu vollständig freiliegen und daher mit dem Sterilisiermittel besprüht werden können.

In einem bevorzugten Ausführungsbeispiel sind die Verschlussaufnahmen mit ihrer Längserstreckung in Richtung der Rotorachse, vorzugsweise parallel oder im Wesentlichen parallel zur Rotorachse orientiert. Dadurch werden bei Drehung des Rotors die in den Verschlussaufnahmen enthaltenen, reihenförmig angeordneten Verschlüsse an den Behandlungsstationen, insbesondere den Sterilisiermittelsprühvorrichtungen vorbeigeführt und können dabei den Sterilisierschritten unterzogen werden. Vorzugsweise sind die Sterilisiermittelsprühvorrichtungen an der Bewegungsbahn der Verschlussaufnahmen innenliegend und/oder außenliegend mit diesen Verschlussaufnahmen oder mit dem Rotor nicht umlaufend vorgesehen.

In einem besonders bevorzugten Ausführungsbeispiel ist einem unteren, offenen Ende der Verschlussaufnahmen gegenüberliegend wenigstens eine mit dem Rotor nicht mitbewegte oder relativ zum Rotor bewegte Verschlussanlagefläche vorgesehen, auf der sich der dieser Verschlussanlagefläche benachbarte Verschluss bei umlaufendem Rotor zur Erzeugung einer von Verschluss zu Verschluss übertragenen Verschluss-Drehbewegung in der Verschlussaufnahme abwälzt. Dabei wird die Drehbewegung des untersten in einer Verschlussaufnahme gelagerten Verschlusses auf die darüber liegenden Verschlüsse übertragen, so dass sich diese paarweise in unterschiedliche Richtungen drehen. Durch diese Drehbewegung wird verhindert, dass Bereiche der Verschlüsse, insbesondere solche, die hinter den Verschlussführungsschienen liegen, nicht mit Sterilisiermittel beaufschlagt werden.

Der Ausdruck "im Wesentlichen" bedeutet im Sinne der Erfindung Abweichungen von jeweils exakten Wert um +/- 10%, bevorzugt um +/- 5% und/oder Abweichungen in Form von für die Funktion unbedeutenden Änderungen. Weiterbildungen, Vorteile und Anwendungsmöglichkeiten der Erfindung ergeben sich auch aus der nachfolgenden Beschreibung von Ausführungsbeispielen und aus den Figuren.

Die Erfindung wird im Folgenden anhand der Figuren an einem Ausführungsbeispiel näher erläutert. Es zeigen:
- Fig. 1: in seitlicher Darstellung eine erfindungsgemäße Vorrichtung zum Sterilisieren von Verschlüssen mit H2O2;
- Fig. 2: eine Schnittdarstellung der Vorrichtung gemäß Figur 1 in einer die Maschinenachse aufnehmenden Schnittebene;
- Fig. 3: eine Schnittdarstellung der Vorrichtung gemäß Figur 1 entlang der Schnittlinie A-A;
- Fig. 4: eine schematische Darstellung zur Erläuterung der verschiedenen ausschließlich in einem einzigen Rotor vorgenommenen Arbeitsschritte zum Sterilisieren von Verschlüssen.

Die in den Figuren allgemein mit 1 bezeichnete Vorrichtung dient zum Sterilisieren und/oder Entkeimen von Verschlüssen 2, beispielsweise von Verschlüssen in Form von Kappen, Schraubkappen, Sportkappen, Flachkappen oder Kronenkorken usw., die zum Verschließen von nicht dargestellten Behältern, z.B. in Form von Flaschen verwendet werden. Die Vorrichtung 1 bildet hierfür bevorzugt ein einer Verschließmaschine zum Verschließen der Behälter vorgeschaltetes Aggregat, aus dem die sterilisierten Verschlüsse 2 unter sterilen bzw. keimfreien Bedingungen der Verschließmaschine zugeführt werden.

Die Vorrichtung umfasst u.a. ein in den Figuren nicht näher dargestelltes Gehäuse oder eine Einhausung, welche eine eine vertikale Maschinen- oder Rotorachse MA umschließende polygonale oder zylindrische Formgebung aufweisen, insbesondere sechseckförmig ausgebildet sein können. Der Innenraum des Gehäuses ist mit Ausnahme von Ein- und Auslässen für die Verschlüsse 2 gegenüber der Umgebung dicht verschlossen, und zwar durch eine die Maschinenachse MA umschließende Umfangswand, durch eine obere Gehäusewand und eine untere Gehäusewand. In der Umfangswand können Inspektionsfenster vorgesehen sein, wobei ebenso eine Ausführung der Umfangswand aus insgesamt transparentem Material möglich ist.

Im Innenraum des Gehäuses ist ein um die vertikale Maschinenachse MA umlaufend antreibbarer Rotor 10 aufgenommen. Bei der dargestellten Ausführungsform ist der Rotor 10 als kreiszylinderförmige, die Maschinenachse MA konzentrisch umschließende Hohltrommel mit einem Trommelmantel mit käfigartiger Struktur ausgeführt, die von einer Vielzahl von Verschlussaufnahmen 11 zur reihenförmigen Aufnahme jeweils einer Vielzahl von Verschlüssen 2 gebildet ist. Die Verschlussaufnahmen 11, die mit ihrer Längserstreckung jeweils parallel oder im Wesentlichen parallel zur Maschinenachse MA orientiert und an ihrem oberen Ende zum Einbringen der Verschlüsse 2 und an ihrem unteren Ende zum Ausbringen der Verschlüsse 2 offen sind, bilden in ihrer Gesamtheit die käfig- oder gitterartige Struktur bzw. den käfigartigen, in sich stabilen Mantel des Rotors 10 und sind hierfür am Umfang des Rotors 10 in gleichmäßigen Winkel- und Teilsabständen um die Maschinenachse MA verteilt vorgesehen. Im Bereich ihrer oberen und unteren Enden sind die Verschlussaufnahmen 11 jeweils an einem konzentrisch zur Maschinenachse MA angeordneten kreisringförmigen oberen bzw. unteren Tragelement 12, 13 befestigt, und zwar derart, dass die unteren, offenen Enden der Verschlussaufnahmen 11 durch das Tragelement 13 nicht abgedeckt sind, vielmehr im Bereich des unteren Tragelementes 13 oder unterhalb dieses Tragelementes 13 freiliegen.

Die Verschlussaufnahmen 11 sind ebenfalls gitterartig ausgeführt und bestehen jeweils aus mehreren voneinander beabstandeten und parallel zur Maschinenachse MA orientierten stangen- oder stabförmigen Verschlussführungsschienen 14, die zwischen sich einen Aufnahmeraum zur reihenförmigen Aufnahme einer Vielzahl von Verschlüssen 2 bilden, und zwar derart, dass die Verschlüsse 2 in jeder Verschlussaufnahme 11 eine sich in einer Achsrichtung parallel zur Maschinenachse MA erstreckende einspurige Verschlussreihe oder -gruppe bilden und in der jeweiligen Verschlussaufnahme 11 möglichst weitgehend freiliegen, d.h. nur an einem geringen Teil ihrer Oberfläche von den Verschlussführungsschienen 14 abgedeckt sind. In vergleichbarer Art und Weise können die Verschlussaufnahmen auch als Frästeile mit entsprechend angepassten Konturen ausgeführt werden.

Weiterhin sind die Verschlussaufnahmen 11 bei der dargestellten Ausführungsform so ausgeführt, dass die Verschlüsse 2 in den Verschlussaufnahmen 11 in Bezug auf die Maschinenachse MA eine vorgegebene Orientierung aufweisen, und zwar in der Form, dass sie mit ihrer Verschluss- oder Kappenachse radial zur Maschinenachse MA und beispielsweise mit ihrer offenen Kappenseite radial nach außen orientiert sind.

Mit dem unteren Tragelement 13 ist der Rotor 10 in einem Lager 15 an der Unterseite der Sterilisiervorrichtung 1 um die Maschinenachse MA drehbar gelagert und mittels eines Antriebs, beispielsweise einem Elektromotor mit Getriebe um die Maschinenachse MA entsprechend dem Pfeil A getaktet, schrittweise umlaufend antreibbar.

Mit dem Rotor 10 nicht umlaufend sind vorzugsweise in dem Innenraum des nicht dargestellten Gehäuses, d.h. in dem Behandlungsraum der Sterilisiervorrichtung 1 Sterilisiermittelsprühvorrichtungen 20, 20' vorgesehen, die vorzugsweise mit ihrer Längserstreckung jeweils parallel zur Maschinenachse MA orientiert sind. Im gezeigten Ausführungsbeispiel sind zwei Paare von Sterilisiermittelsprühvorrichtungen 20, 20' vorgesehen, wobei jeweils eine erste Sterilisiermittelsprühvorrichtung 20 des Sterilisiermittelsprühvorrichtungspaares radial innenliegend und eine zweite Sterilisiermittelsprühvorrichtung 20' radial außenliegend in Bezug auf die Verschlussaufnahmen 11 vorgesehen sind.

Die Sterilisiermittelsprühvorrichtungen 20, 20' weisen im gezeigten Ausführungsbeispiel einen ersten, rohrförmig ausgebildeten Sprühvorrichtungsabschnitt 20.1, 20.1' auf, der parallel zur Maschinenachse MA verläuft. Dabei ist die Sterilisiermittelsprüheinrichtung 20, 20' vorzugsweise als Ringleitung ausgeformt, so dass eine gleichmäßige Versorgung aller seitlichen Abgänge sicherstellt ist.

An diesem ersten Sprühvorrichtungsabschnitt 20.1, 20.1' sind eine Vielzahl von Auslässen 21 für das Sterilisiermittel vorgesehen, die durch die freiendseitigen Enden von mit dem ersten Sprühvorrichtungsabschnitt 20.1, 20.1', fluiddicht verbundenen Düsen 22 gebildet werden. Die Düsen 22 sind hierbei vorzugsweise radial in Bezug auf den Rotor 10 mit Ihrer Längserstreckung ausgerichtet und stehen derart von den ersten Sprühvorrichtungsabschnitten 20.1, 20.1' ab, das die Auslässe 21 in unmittelbarer Nähe zu den bei Drehung des Rotors 10 vorbeibewegten Verschlussaufnahmen 11 zu liegen kommen.

Vorzugsweise sind die Düsen 22 einer radial innen liegenden Sprühmittelvorrichtung 20 und einer radial außen liegenden Sterilisiermittelsprühvorrichtung 20' in radialer Richtung zentriert zueinander ausgerichtet, sodass ein in einer Verschlussaufnahme 11 aufgenommener Verschluss 2 gleichzeitig vorder- und rückseitig mit dem Sterilisiermittel besprüht bzw. beaufschlagt werden kann. Bevorzugt sind die Düsen 22 freiendseitig trichterartig ausgebildet, sodass eine vollflächige vorder- bzw. rückseitige Beaufschlagung der Verschlüsse 2 mit Sterilisiermittel ermöglicht wird.

Bei einer alternativen Variante, sind die Düsen an ihrem weiten Ende kugelförmig gewölbte und mit einer Vielzahl von Bohrungen versehene sein, wodurch ausströmendes Sterilisiermittel großflächig verteilt wird.

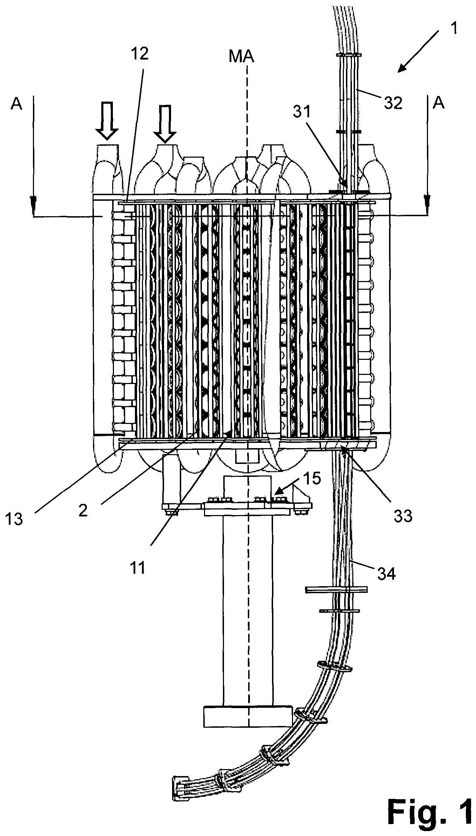

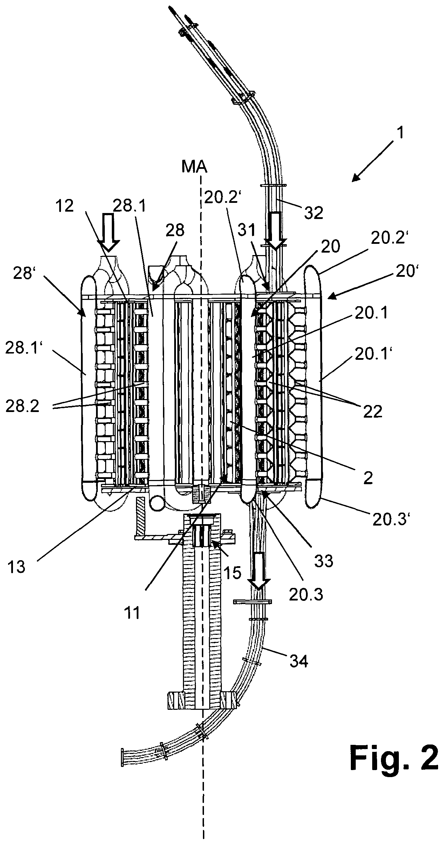

Wie in den Figuren 1 und 2 erkennbar, erstrecken sich die Sterilisiermittelsprühvorrichtungen 20, 20' zumindest im Wesentlichen über die gesamte Längserstreckung der Verschlussaufnahmen 11 und weisen eine Vielzahl von radial von den ersten Sprühvorrichtungsabschnitten 20.1, 20.1' abstehende Düsen 22 auf. Der Abstand der Düsen 22 ist hierbei vorzugsweise derart dimensioniert, dass er dem Durchmesser der in den Verschlussaufnahmen 11 aufgenommenen Verschlüssen 2 entspricht. Damit kommt jeweils eine Düse 22 der Sterilisiermittelsprühvorrichtungen 20, 20' innen- bzw. außenseitig in Bezug auf die Verschlussaufnahme 11 gegenüber den darin aufgenommenen Verschlüssen 2 zu liegen, sodass jedem Verschluss 2 jeweils ein eigenes Düsenpaar zugeordnet ist, das ein gezieltes Beaufschlagen des Verschlusses 2 mit dem Sterilisiermittel ermöglicht.

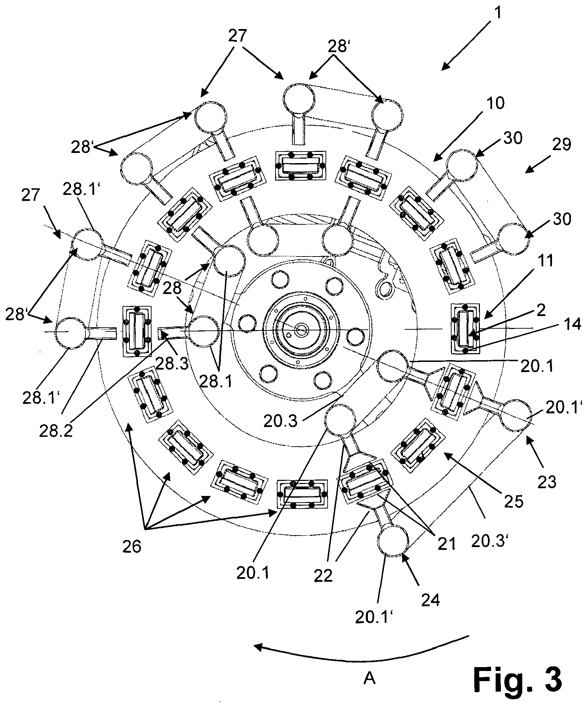

Wie aus Figur 3 erkennbar ist, sind in Dreh- oder Umlaufrichtung des Rotors A innen- bzw. außenumfangsseitig mehrere Behandlungsstationen zum Aufbringen des Sterilisiermittels vorgesehen. Eine erste und eine zweite Applikationsstation 23, 24 sind vorgesehen, an denen jeweils zwei einander gegenüberliegende, zuvor beschriebene Sterilisiermittelsprühvorrichtungen 20, 20' angeordnet sind. Die ersten Sprühvorrichtungsabschnitte 20.1' der radial außenliegend angeordneten Sterilisiermittelsprühvorrichtungen 20' sind ober- bzw. unterseitig durch bogen- bzw. jochartig ausgebildete zweite Sprühvorrichtungsabschnitte 20.2', 20.3' verbunden, wobei vorzugsweise jeweils über den oberseitig angeordneten zweiten Sprühvorrichtungsabschnitt 20.2' eine zentrale Sterilisiermittelzuführung erfolgt. Ebenso sind die ersten Sprühvorrichtungsabschnitte 20.1 der radial innenliegend angeordneten Sterilisiermittelsprühvorrichtungen 20 der ersten und zweiten Applikationsstation 23, 24 ober- bzw. unterseitig über jochartig ausgebildete zweite Sprühvorrichtungsabschnitte 20.2, so dass eine Ringleitung ausgebildet wird, wodurch eine sehr gleichmäßige Beaufschlagung der seitlichen Auslässe erreicht wird.

Über die voneinander getrennten Applikationsstationen 23, 24 können die in einer Verschlussaufnahme 11 aufgenommenen Verschlüsse 2 zeitlich nacheinander mit Sterilisiermittel beaufschlagt werden, und zwar zu einem ersten Zeitpunkt durch die erste Applikationsstation 23 und nach einer getakteten Drehung des Rotors 10 zu einem späteren, zweiten Zeitpunkt durch die weitere, zweite Applikationsstation 24. Durch die in festen Winkel- und Teilungsabständen um die Maschinenachse MA angeordneten Verschlussaufnahmen 11 wird der Winkelbetrag festgelegt, um den der Rotor 10 bei dem schrittweisen, intermittierenden Drehbewegung um die Maschinenachse MA rotiert. Die erste und zweite Applikationsstation 23, 24 sind im gezeigten Ausführungsbeispiel um zwei Winkel- oder Teilungsabstände zueinander beabstandet, sodass die in einer Verschlussaufnahme 11 aufgenommen Verschlüsse 2, die in der ersten Applikationsstation 23 behandelt wurden, bei einer darauf folgenden getakteten Drehung des Rotors 10 im darauf folgenden Behandlungstakt zwischen der ersten und zweiten Applikationsstation 23, 24 zu liegen kommt, an der keinerlei Behandlung stattfindet. Hierbei handelt es sich vielmehr um eine Verweilstation 25, an der das auf die Verschlüsse 2 in der ersten Applikationsstation 23 aufgebrachte Sterilisiermittel einwirken kann. Bei einer weiteren getakteten Drehung des Rotors 10 gelangen die zuvor an der Verweilstation 25 befindlichen Verschlüsse 2 an die zweite Applikationsstation 24, um dort erneut mit Sterilisiermittel beaufschlagt zu werden. Damit wird eine zweistufige unmittelbare Beaufschlagung der einzelnen Verschlüsse mit Sterilisiermittel vorzugsweise von beiden Verschlussseiten her erreicht. Alternativ kann natürlich vorgesehen werden, mehrere Applikationsstationen ohne eine dazwischenliegende Verweilstation anzuordnen.

In Umlaufrichtung an die zweite Applikationsstation 24 anschließend folgen vorzugsweise weitere Verweilstationen 26, im gezeigten Ausführungsbeispiel vier Verweilstationen 26. Diese dienen nach der erneuten Beaufschlagung der Verschlüsse 2 mit dem Sterilisiermittel wiederum der Optimierung des Entkeimungs- und/oder Sterilisierverfahrens durch eine verlängerte Einwirkzeit des Sterilisiermittels. An die Verweilstationen 26 anschließend folgen in Umlaufrichtung A Aktivierungs- und Trocknungsstationen 27, und zwar im gezeigte Ausführungsbeispiel sechs derartige Aktivierungs- und Trocknungsstationen 27. Damit werden die Verschlüsse 2 in sechs aufeinanderfolgenden Taktzeiten getrocknet und aktiviert. Zur Trocknung der Verschlüsse 2 werden diese mit einem heißen Aktivierungs- und Trocknungsmedium, bevorzugt mit erhitzter, steriler Luft beaufschlagt. Durch diese Beaufschlagung werden die Verschlüsse 2 nicht nur einem Trocknungsvorgang unterzogen, sondern das im Sterilisationsmedium enthaltene Wasserstoffperoxid (H₂O₂) wird bei dieser Beaufschlagung mit erhitzter, steriler Luft in bekannter Weise aktiviert und zum Zerfall angeregt, sodass die Verschlüsse 2 mit hoher Qualität sterilisiert werden.

An den Aktivierungs- und Trocknungsstationen 27 sind jeweils Trocknungsvorrichtungen 28 vorgesehen, mittels denen das Trocknungsmedium den in den Verschlussaufnahmen 11 befindlichen Verschlüssen 2 gezielt zugeführt wird. Hierbei sind den einzelnen Aktivierungs- und Trocknungsstationen 27 radial außenseitig jeweils angeordnete Trocknungsvorrichtungen 28' zugeordnet. Zudem sind radial innenseitig ebenfalls zumindest teilweise den Aktivierungs- und Trocknungsstationen 27 zugeordnete Trocknungsvorrichtungen 28 vorgesehen, sodass zumindest teilweise an den Aktivierungs- und Trocknungsstationen 27 eine beidseitige Beaufschlagung der Verschlüsse 2 mit dem Trocknungsmedium erfolgt. Die Trocknungsvorrichtungen 28 sind im Wesentlichen gleich zu den Sterilisiermittelsprühvorrichtungen 20, 20', ausgebildet und weisen jeweils einen rohrförmigen ersten Trockenvorrichtungsabschnitt 28.1, 28.1', auf, der jeweils parallel zur Maschinen- und Rotorachse MA ausgerichtet ist. An diesem ersten Trocknungsvorrichtungsabschnitt 28.1, 28.1', sind seitlich, radial abstehende Düsen 28.2 vorgesehen, über die das in dem ersten, rohrförmigen Trocknungsvorrichtungsabschnitt 28.1, 28.1', geführte Aktivierungs- und Trocknungsmedium über Auslässe 28.3 gezielt bzw. gerichtet auf die Verschlüsse austreten kann. Die Düsen 28.2 der Trocknungsvorrichtungen 28, 28', die sich an einer Aktivierungs- und Trocknungsstation 27 gegenüberliegen, sind in Bezug auf ihre Längsachsen achsgleich ausgerichtet und verlaufen radial in Bezug auf die Maschinen- und Rotorachse MA. Dadurch wird eine gezielte Beaufschlagung des Verschlusses 2 vorder- und rückseitig mit dem Trocknungsmedium erreicht.

Des Weiteren sind an den Trocknungsvorrichtungen 28, 28', jeweils eine Vielzahl derartiger Düsen 28.2 vorgesehen, die in Richtung der Längsachse des ersten Trocknungsvorrichtungsabschnitts 28.1, 28.1' derart zueinander beabstandet sind, dass jeweils eine Düse 28.2 vorzugsweise zentriert gegenüber einem in der Verschlussaufnahme 11 in der Aktivierungs- und Trocknungsstation 27 befindlichen Verschluss 2 zu liegen kommt. Vorzugsweise ist jedem Verschluss 2, der in der jeweiligen Verschlussaufnahme 11 in der Aktivierungs- und Trocknungsstation 27 gelegen ist, jeweils eine Düse 28.2 bzw. ein einander gegenüberliegendes Düsenpaar zugeordnet, sodass jeder Verschluss 2 der Verschlussreihe gezielt einzeln mit dem Aktivierungs- und Trocknungsmedium beaufschlagt wird.

An die Aktivierungs- und Trocknungsstationen 27 in Umlaufrichtung A anschließend sind zumindest eine, im gezeigten Ausführungsbeispiel zwei Kühlstationen 29 vorgesehen, an denen eine Kühlung der zuvor sterilisierten Verschlüsse 2 durch Beaufschlagung mit steriler Luft bewirkt wird. An den Kühlstationen 29 ist hierbei vorzugsweise außenumfangsseitig eine Kühlvorrichtung 30 vorgesehen, die im gezeigten Ausführungsbeispiel baugleich mit der Trocknungsvorrichtung 28, 28' ausgebildet ist. Über diese Kühlvorrichtung 30 können die einzelnen reihenförmig in der Verschlussaufnahme 11 aufgenommenen Verschlüsse gleichzeitig gezielt mit dem Kühlmedium, insbesondere gekühlter, steriler Luft beaufschlagt werden.

In Umlaufrichtung vor der ersten Applikationsstation 23 bzw. nach der Kühlstation 29 ist über der Bewegungsbahn der Verschlussaufnahmen 11 eine Verschlussaufgabe 31 vorgesehen, die von einem unteren Ende einer äußeren Verschlussförderstrecke 32 gebildet ist und der die zu sterilisierenden Verschlüsse 2 über diese Verschlussförderstrecke 32 zugeführt werden. In der Verschlussförderstrecke 32 weisen die Verschlüsse 2 bereits die ihrer Orientierung in den Verschlussaufnahmen 11 entsprechende Orientierung auf. Im Bereich der Unterseite des Gehäuses 3 ist unterhalb der Bewegungsbahn der Verschlussaufnahmen 11 eine Verschlussabgabe 33 vorgesehen, die im Wesentlichen von dem Einlass oder von einem oberen offenen Ende einer Verschlussförderstrecke 34 gebildet ist, welche mit ihrem offenen Ende unterhalb der Bewegungsbahn der Verschlussaufnahmen 11 des Rotors 10 angeordnet ist und dort die Verschlussabgabe 33 bildet. Über die Verschlussförderstrecke 34 werden die sterilisierten Verschlüsse 2 der nicht dargestellten Verschließmaschine keimfrei zugeführt.

Bei der dargestellten Ausführungsform sind die Verschlussförderstrecken 32 und 34 jeweils von mehreren, die Verschlüsse 2 zwischen sich aufnehmenden und führenden Führungsschienen gebildet. Weiterhin weisen bei der dargestellten Ausführungsform die Verschlussförderstrecken 32 und 34 zumindest in der Nähe des Rotors 10 jeweils einen vertikalen Verlauf auf, sodass die Verschlüsse 2 in den Verschlussförderstrecken 32 und 34 allein schon aufgrund ihrer Schwerkraft bewegt bzw. gefördert werden. Um allerdings ein erneutes Verkeimen der sterilisierten Verschlüsse 2 auf der Verschlussförderstrecke 34 zu vermeiden ist diese in einer nicht dargestellten Ummantelung oder Einhausung aufgenommen, die bevorzugt mit einem Überdruck eines sterilen gas- und/oder dampfförmigen Mediums, beispielsweise mit dem Überdruck steriler Luft beaufschlagt ist.

Ein besonderer Vorteil dieser hier beschriebenen Sterilisierungsvorrichtung in den unterschiedlichen Ausgestaltungen ist, dass durch den Schrittbetrieb die Verweilzeit vor den einzelnen Behandlungsstufen sehr einfach verändert und gesteuert werden kann, so dass bspw. für Verschlüsse mit einer sehr komplexen inneren Struktur längere Verweilzeiten vorgesehen werden, als für einfache kleine Verschlüsse.

Die Verschlussaufgabe 31 und die Verschlussabgabe 33 sind bezogen auf die Maschinenachse MA in einem Winkelabstand, der einem Teilungsabstand der Verschlussaufnahmen 11 am Rotor 10 oder bevorzugt einem Vielfachen dieses Teilungsabstandes entspricht, so angeordnet, dass immer dann, wenn sich eine Verschlussaufnahme 11 mit ihrem oberen offenen Ende in der Stillstandsphase der Drehbewegung des Rotors 10 an der Verschlussaufgabe 31 befindet, sich eine andere Verschlussaufnahme 11 mit ihrem unteren offenen Ende an der Verschlussabgabe 33 befindet. Weiterhin ist die Verschlussabgabe 33 bezogen auf die Umlaufrichtung A des Rotors 10 um einen möglichst großen Winkelbetrag, beispielsweise um einen Winkelbetrag etwas kleiner als 360°, z.B. um einen Winkelbetrag von 330° oder von etwa 330° von der Verschlussaufgabe 31 beabstandet, um eine möglichst lange Behandlungsstrecke und damit auch bei hohen Leistungen der Vorrichtung 1, d.h. bei einer hohen Anzahl der je Zeiteinheit mit dieser Vorrichtung 1 sterilisierten Verschlüssen 2 bzw. bei einer entsprechend hohen Drehgeschwindigkeit des Rotors 10 eine möglichst lange Behandlungsdauer für das Entkeimen bzw. Sterilisieren der Verschlüsse 2 zu erreichen, und zwar bei reduzierten Durchmesser des Rotors 10 und kompakter Bauform der Vorrichtung 1 insgesamt.

Beispielsweise ist die Verschlussaufgabe 31 oberhalb der ersten Applikationsstation 23 angeordnet, wohingegen die Verschlussabgabe 33 bezogen auf die Umlaufrichtung A in einem Winkel- bzw. Teilungsabstand vor der Applikationsstation 23 angeordnet ist, oder aber die Verschlussaufgabe 31 ist vorzugsweise bezogen auf die Umlaufrichtung A in einem Winkel- bzw. Teilungsabstand vor der Applikationsstation 23 angeordnet, wobei sich die Verschlussabgabe 33 dann an der bezogen auf die Umlaufrichtung A letzten Kühlvorrichtung 30 befindet. Diese zuletzt genannte Ausführung hat den Vorteil, dass auch bereits an der bezogen auf die Umlaufrichtung A ersten Applikationsstation 23 auf jeden Fall die volle Stillstandszeit der getakteten oder schrittweisen Bewegung des Rotor 10 für das gezielte Aufbringen des Sterilisationsmediums (H₂O₂-Aerosol) auf die Verschlüsse 2 zu Verfügung steht.

Mit einem nicht näher dargestellten Antrieb wird der Rotor 10 getaktet um die Maschinenachse MA angetrieben und zwar derart, dass der Rotor 10 in jeder Bewegungsphase der getakteten Drehbewegung einen dem Teilungsabstand zweier Verschlussaufnahmen 11 entsprechenden Drehschritt ausführt und sich in jeder Stillstandsphase der getakteten Drehbewegung eine leere Verschlussaufnahme 11 unterhalb der Verschlussaufgabe 31 befindet, über die dann aus der Verschlussförderstrecke 32 die Verschlussaufnahme 11 vollständig mit Verschlüssen 2 gefüllt wird. Mit der getakteten Drehbewegung des Rotors 10 werden die jeweils mit den Verschlüssen 2 gefüllten Verschlussaufnahmen 11 entlang der Behandlungsstationen der Behandlungsstrecke bewegt und dabei in den Stillstandsphasen des Rotors 10 an den einzelnen Stationen behandelt und dabei entkeimt.

Immer dann, wenn eine Verschlussaufnahme 11 des Rotors 10 die Verschlussabgabe 33 erreicht hat, fallen sämtliche, sterilisierten Verschlüsse 2 dieser Verschlussaufnahme in die Verschlussförderstrecke 34, über die die entkeimten bzw. sterilisierten Verschlüsse 2 dann der weiteren Verwendung zugeführt werden.

Die Sterilisiermittelsprühvorrichtungen 20, 20' die Trocknungsvorrichtungen 28, 28' sowie die Kühlvorrichtungen 30 werden während des Betriebs der Vorrichtung 1 intermittierend betrieben, d.h. synchron mit der intermittierenden oder schrittweise Drehbewegung des Rotors 10 getaktet angesteuert, so dass jeweils nach der Drehung des Rotors in dessen Stillstandsphase das Sterilisiermedium, das Trocknungsmedium bzw. des Kühlmedium appliziert wird.

Eine Besonderheit der Vorrichtung 1 besteht darin, dass sämtliche Behandlungsschritte der Sterilisierung der Verschlüsse 2, d.h. die Beaufschlagung mit dem Sterilisierungsmittel bzw. mit dem H₂O₂-Aerosol, die Aktivierung des Sterilisierungsmittels und die Trocknung sowie bevorzugt auch die Kühlung der Verschlüsse 2 in dem Rotor 10 erfolgen, mit dem die in den Verschlussaufnahmen 11 angeordneten Verschlüsse 2 entlang der zwischen der Verschlussaufgabe 31 und der Verschlussabgabe 33 gebildeten Behandlungsstrecke an den einzelnen Behandlungszonen vorbei bewegt werden. Eine weitere Besonderheit der Vorrichtung 1 besteht auch darin, dass die Behandlungsmedien gezielt, d.h. strahlförmig gerichtet auf die Verschlüsse 2 innen und außen aufgebracht werden, was nicht nur für das Sterilisierungsmediums (H₂O₂-Aerosol), sondern auch für das heiße Aktivierungs- und Trocknungsmedium (Aktivierungs- und/oder Trocknungsstationen 27) und das Kühlmedium (Kühlstation 29) gilt.

In einer alternativen Ausführungsform wird statt H₂O₂ als Sterilisierungsmittel ein anderes flüssiges oder dampfförmiges Sterilisierungsmittel bzw. Aerosol verwendet, insbesondere Oxonia oder Peressigsäure. Natürlich kann auch ein gasförmiges Sterilisierungsmittel zum Einsatzkommen, wie bspw. Ozon, wobei dann Behandlungsschritte entsprechend angepasst werden.

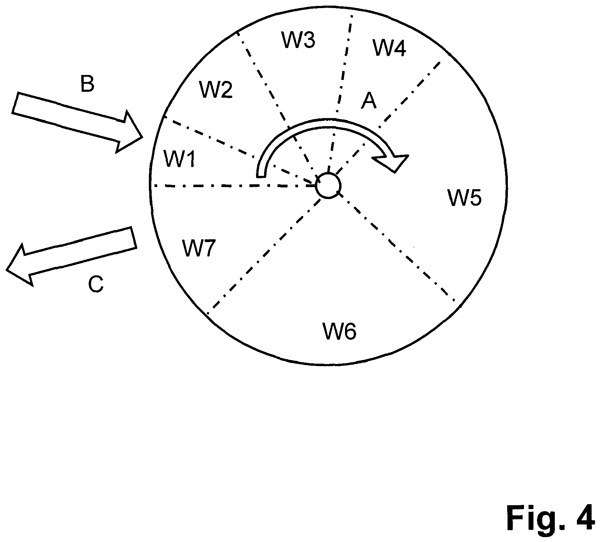

Die Figur 4 zeigt nochmals schematisch die einzelnen Behandlungsschritte beim Sterilisieren der Verschlüsse 2. Mit W1 - W7 sind in dieser Figur Winkelbereiche der Drehbewegung des Rotors 10 bezeichnet. Im ersten Winkelbereich W1 erfolgt entsprechend dem Pfeil B das Zuführen der Verschlüsse 2 an die jeweilige Verschlussaufnahme 11 an der Verschlussaufgabe 31. Auf dem folgenden Winkelbereich W2 erfolgt die erste gezielte und gesteuerte Beaufschlagung der Verschlüsse 2 an ihrer Innen- und Außenseite mit dem H₂O₂-Aerosol. In dem anschließenden Winkelbereich W3 erfolgt dann an der Verweilstation 25 ein Einwirken des H₂O₂-Aerosols auf die Verschlüsse 2. In dem anschließenden Winkelbereich W4 erfolgt eine erneute Beaufschlagung der Verschlüsse 2 an ihrer Innen- und Außenseite mit dem H₂O₂-Aerosol. Bei der beschriebenen Vorrichtung 1 entsprechen die Winkelbereiche W1, W2, W3 und W4 jeweils einen Taktschritt der getakteten Drehbewegung des Rotors 10.

In dem auf den Winkelbereich W4 folgenden Winkelbereich W5 erfolgt ein weiteres Einwirken des Sterilisiermittels auf die Verschlüsse 2 an den Verweilstationen 26, wobei der Winkelbereich W5 bei der Sterilisiervorrichtung 1 insgesamt vier Taktschritten der getakteten Bewegung des Rotors entspricht.

Auf dem Winkelbereich W6 erfolgt die Beaufschlagung der Verschlüsse 2 innen und außen mit dem heißen Aktivierungs- und Trocknungsmedium, bevorzugt mit der erhitzten, sterilen Luft. Bei der Vorrichtung 1 entspricht der Winkelbereich W6 insgesamt sechs Taktschritten der getakteten Bewegung des Rotors 10. An dem folgenden Winkelbereich W7, der bei der Vorrichtung 1 Zweitaktschritten der Drehbewegung des Rotors 10 entspricht, erfolgt dann an der Kühlstation 29 das Abkühlen sowie auch das Abführen der sterilisierten Verschlüsse 2 an der Verschlussabgabe 33 entsprechend dem Pfeil C.

Um sicherzustellen, dass auch bei veränderten Verschlussdurchmessern, z.B. von 38mm Durchmesser auf Verschlüsse mit 28mm Durchmesser, die Auslässe und Düsen immer im Wesentlichen mittig bzw. achsgleich zu den Verschlüssen angeordnet sind, d.h. der Fluidstrahl (Sterilisierungsmittel, Aktivierungs- und Trocknungsmittel, Kühlluft/-gas, etc.) mittig und/oder senkreckt in dem jeweiligen Verschluss auftrifft, sind Auslässe oder Ventile in Führungen gehalten und in ihrem Abstand zueinander veränderlich. Alternativ sind die vertikal benachbarten Auslässe in einem länglichen Tragelement angeordnet, welches als Formatteil an den vertikalen Schenkeln der entsprechenden Behandlungsstation angebracht bzw. abgenommen werden kann.

Bedarfsweise kann bei den beschriebenen Ausführungsformen der Vorrichtung oder des Verfahrens das flüssige H₂O₂ oder die H₂O₂-haltige Lösung in oder durch entsprechende Ein- oder Zweistoffdüsen zersteubt und auf die Verschlüsse geleitet werden. Hierzu sind die Auslässen (21) entsprechend konstruiert oder umfassen solche Ein- oder Zweistoffdüsen und die Zuleitungen und Sprühvorrichtung/- abschnitte) sind entsprechend angepasst.

Die Erfindung wurde voranstehend an einem Ausführungsbeispiel beschrieben. Es versteht sich, dass hierin auch ein Sterilisierungsverfahren u.a. unter Nutzung vorgenannter Vorrichtung umfasst ist und weiterhin zahlreiche Änderungen sowie Abwandlungen möglich sind, ohne den Schutzumfang der nachfolgenden Ansprüche zu verlassen. Hinsichtlich des Sterilisierungsverfahrens für Verschlüsse ist dabei wesentlich, dass Verschlüsse vertikal in Verschlussaufnahme einer Sterilisationsvorrichtung geleitet werden, und nachfolgend in dieser gestapelten und gleichausgerichteten Position, im Wesentlichen ohne weitere Relativbewegung untereinander oder zu dem Aufnahmeelement, als Gruppe schrittweise (in Winkelschritten) bewegt bzw. relativ zu den mehreren Behandlungsstationen wie o.g. positioniert, behandelt und dann weitergeleitet werden.

### Bezugszeichenliste

- 1: Sterilisiervorrichtung
- 2: Verschluss

- 10: Rotor
- 11: Verschlussaufnahme
- 12: oberes Tragelement
- 13: unteres Tragelement
- 14: Verschlussführungsschiene
- 15: Lager
- 16: Verschlussanlagefläche

- 20, 20': Sterilisiermittelsprühvorrichtung
- 20.1, 20.1': erster Sprühvorrichtungsabschnitt
- 20.2, 20.2': zweiter Sprühvorrichtungsabschnitt
- 20.3, 20.3': dritter Sprühvorrichtungsabschnitt
- 21: Auslass
- 22: Düse
- 23: erste Applikationsstation
- 24: zweite Applikationsstation
- 25: Verweilstation
- 26: Verweilstation
- 27: Aktivierungs- und/oder Trocknungsstation
- 28, 28': Trocknungsvorrichtung
- 28.1, 28.1': erster Trocknungsvorrichtungsabschnitt
- 28.2: Düse
- 28.3: Auslass
- 29: Kühlstation
- 30: Kühlvorrichtung
- 31: Verschlussaufgabe
- 32: Verschlussförderstrecke
- 33: Verschlussabgabe
- 34: Verschlussförderstrecke

- A: Dreh- oder Umlaufrichtung des Rotors 10
- B: Zuführung der Verschlüsse 2
- C: Entnahme der Verschlüsse 2
- W1 - W6: Winkelbereich der Drehbewegung des Rotors 10
- MA: Maschinenachse

## Patentansprüche

1. Vorrichtung zum Sterilisieren und/oder Entkeimen von Verschlüssen (2) zum Verschließen von Flaschen oder dergleichen Behältern, insbesondere zum Sterilisieren von kappenartigen Verschlüssen, mit einem Transportsystem zum Bewegen der Verschlüsse (2) durch mehrere Behandlungszonen, in der die Verschlüsse (2) mindestens sterilisiert sowie aktiviert und getrocknet werden, wobei das Transportsystem in der wenigstens einen Behandlungszone wenigstens einen um eine Maschinen- oder Rotorachse (MA) umlaufend antreibbaren Rotor (10) mit einer Vielzahl von am Umfang dieses Rotors (10) ausgebildeten, vertikal ausgerichtete Verschlussaufnahmen (11) aufweist, mittels denen eine Mehrzahl von Verschlüssen (2) als Gruppe gestapelt aufnehmbar und in Umfangsrichtung (A) umlaufend durch mehrere Behandlungszonen auf einer Behandlungsstrecke zwischen einer Verschlussaufgabe (31) und einer Verschlussabgabe (33) bewegbar sind,
wobei der wenigstens eine Rotor (10) hohltrommel- oder hohlzylinderartig mit einem von einer Vielzahl von Verschlussaufnahmen (11) gebildeten käfigartigen Trommelmantel ausgeführt ist, wobei die Verschlussaufnahmen (11) ihrerseits bevorzugt jeweils käfigartig mit sich in Längsrichtung der jeweiligen Verschlussaufnahme (11) erstreckenden stab- und/oder stangenförmigen Verschlussführungsschienen (14) ausgebildet sind, zwischen denen die Verschlüsse (2) in der jeweiligen Verschlussaufnahme (11) aufgenommen sind, und wobei die Vorrichtung weiterhin ein Gehäuse oder eine Einhausung umfasst, welche eine die vertikale Maschinen- oder Rotorachse (MA) umschließende polygonale oder zylindrische Formgebung aufweisen, wobei der Innenraum des Gehäuses mit Ausnahme von Ein- und Auslässen für die Verschlüsse (2) gegenüber der Umgebung durch eine die Maschinenachse (MA) umschließende Umfangswand und durch eine obere Gehäusewand und eine untere Gehäusewand dicht verschlossen ist, und wobei im Innenraum des Gehäuses der um die vertikale Maschinenachse (MA) umlaufend antreibbare Rotor (10) aufgenommen ist,
**dadurch gekennzeichnet, dass**
an der wenigstens einen Behandlungsstrecke zumindest eine Applikationsstation (23, 24) mit einer auf zumindest eine Verschlussaufnahme (11) gerichteten Sterilisiermittelsprühvorrichtung (20, 20') zum Beaufschlagen der einzelnen in der Verschlussaufnahme (11) aufgenommenen Verschlüsse (2) mit einem Sterilisiermittel vorgesehen ist, wobei die Sterilisiermittelsprühvorrichtung (20, 20') jeweils eine Vielzahl von Auslässen (21) aufweist, wobei die aufeinanderfolgenden Auslässe (21) jeweils um einen Verschlussdurchmesser oder im Wesentlichen einen Verschlussdurchmesser in Richtung der Längserstreckung der Verschlussaufnahmen (11) beabstandet sind, und wobei
in Umlaufrichtung (A) auf die zumindest eine Applikationsstation (23, 24) folgend zumindest eine Aktivierungs- und Trocknungsstation (27) vorgesehen ist, an welchen die Verschlüsse mit einem Trocknungs- und Aktivierungsmedium getrocknet werden können.

2. Vorrichtung nach Anspruch 1, **dadurch gekennzeichnet, dass** an der zumindest einen Applikationsstation (23, 24) zumindest zwei einander gegenüberliegende Sterilisiermittelsprühvorrichtungen (20, 20') vorgesehen sind, die zum Beaufschlagen der einzelnen in einer Verschlussaufnahme (11) aufgenommenen Verschlüsse (2) mit dem Sterilisiermittel von gegenüberliegenden Seiten ausgebildet sind.

3. Vorrichtung nach Anspruch 1 oder 2, **dadurch gekennzeichnet, dass** in Umlaufrichtung (A) des Rotors (10) aufeinander folgend mehrere Applikationsstationen (23, 24) zum Beaufschlagen der in den Verschlussaufnahmen (11) aufgenommenen Verschlüsse (2) mit dem Sterilisiermittel vorgesehen sind.

4. Vorrichtung nach Anspruch 3, **dadurch gekennzeichnet, dass** die Verschlussaufnahmen (11) in festen Winkel- oder Teilungsabständen um die Maschinen- oder Rotorachse (MA) verteilt angeordnet sind und die aufeinanderfolgenden Applikationsstationen (23, 24) zueinander um mindestens zwei Winkel- oder Teilungsabstände zueinander beabstandet sind.

5. Vorrichtung nach einem der Ansprüche 3 oder 4, **dadurch gekennzeichnet, dass** die einzelnen Auslässe (21) der Sterilisiermittelsprühvorrichtung (20, 20') und/oder die Sterilisiermittelsprühvorrichtungen (20, 20') unterschiedlicher Applikationsstationen (23, 24) mit einer zentralen Sterilisiermittelzuführung verbunden sind.

6. Vorrichtung nach Anspruch 4, **dadurch gekennzeichnet, dass** in Umlaufrichtung auf die zumindest eine Applikationsstation (23, 24) folgend vor der zumindest einen Aktivierungs- und Trocknungsstation (27) zumindest eine Verweilstation (26) vorgesehen ist.

7. Vorrichtung nach Anspruch 4 oder 5, **dadurch gekennzeichnet, dass** in Umlaufrichtung (A) auf die zumindest eine Aktivierungs- und/oder Trocknungsstation (27) folgend zumindest eine Kühlstation (29) vorgesehen ist.

8. Vorrichtung nach einem der vorhergehenden Ansprüche, **dadurch gekennzeichnet, dass** die Verschlussaufnahmen (11) mit ihrer Längserstreckung parallel oder im Wesentlichen parallel zur Rotorachse (MA) orientiert sind.

9. Vorrichtung nach einem der vorhergehenden Ansprüche, **dadurch gekennzeichnet, dass** die Sterilisiermittelsprühvorrichtungen (20, 20') an der Bewegungsbahn der Verschlussaufnahmen (11) innenliegend und/oder außenliegend vorgesehen sind, und wobei diese mit diesen Verschlussaufnahmen (11) oder mit dem Rotor (10) nicht umlaufen.

10. Vorrichtung nach einem der vorhergehenden Ansprüche, gekennzeichnet eine oder mehrere zentrale Gasleitungen und/oder Gasleitungselemente sowie hiermit verbundene Förder-, Steuer- und/oder Regelvorrichtungen, zum Gas- und Atmosphärenmanagement im Inneren der Vorrichtung.

11. Verfahren zur Sterilisierung von Verschlüssen, **dadurch gekennzeichnet, dass** eine Vorrichtung nach einem der vorhergehenden Ansprüche verwendet wird.

## Claims

1. Device for the sterilisation and/or disinfection of seals (2) for the sealing of bottles or similar containers, in particular for the sterilisation of cap-like seals, with a transport system for moving the seals (2) through several treatment zones, in which the seals (2) are at least sterilised as well as being activated and dried, wherein the transport system comprises in the at least one treatment zone at least one rotor (10), driven such as to rotate about a machine axis or rotor axis (MA), with, formed at the circumference of this rotor (10), a multiplicity of vertically aligned seal holders (11), by means of which a multiplicity of seals (2) can be taken up stacked as a group, and can be moved in a circumferential direction (A), circulating through several treatment zones on a treatment path between a seal inlet (31) and a seal outlet (33) wherein
the at least one rotor (10) is configured as a hollow drum or hollow cylinder, with a cage-like drum casing formed from a multiplicity of seal holders (11), wherein the seal holders (11) in their turn preferably in each case formed in cage fashion, with seal guide rails (14) in bar and/or rod form extending in the longitudinal direction of the respective seal holder (11), between which the seals (2) are taken up in the respective seal holder (11), and wherein
the device further comprises a housing or enclosure, which exhibits a polygonal or cylindrical shape surrounding a vertical machine axis or rotor axis (MA), wherein the interior space of the housing is closed with sealing effect, with the exception of inlets and outlets for the seals (2), against the surrounding environment by a circumferential wall surrounding the machine axis (MA) and by an upper housing wall and a lower housing wall, and wherein, in the interior space of the housing the rotor (10) is accommodated, which can be driven such as to circulate about the vertical machine axis (MA), **characterised in that**
provided at the at least one treatment segment is at least one application station (23, 24), with a sterilising agent spray device (20, 20') directed onto at least one seal holder (11), for imposing on the individual seals (2) taken up in the seal holder (11) a sterilising agent, wherein the sterilising agent spray device (20, 20') comprises in each case a multiplicity of outlets (21), wherein the outlets (21) following one another in sequence are in each case separated from one another by one seal diameter or essentially one seal diameter in the direction of the longitudinal extension of the seal holders (11), and wherein,
in the circulating direction (A), following the at least one application station (23, 24), at least one activating and drying station (27) is provided, at which the seals can be dried with a drying medium and activating medium.

2. Device according to claim 1, **characterised in that**, provided at the at least one application station (23, 24) are at least two mutually opposed sterilising agent spray devices (20, 20'), which are configured for imposing the sterilising agent on the seals (2), individually taken up and held in a seal holder (11), from opposite sides.

3. Device according to claim 1 or 2, **characterised in that**, in the circulating direction (A) of the rotor (10), following one another, several application stations (23, 24) are provided, for imposing the sterilising agent onto the seals (2) held in the seal holders (11).

4. Device according to claim 3, **characterised in that** the seal holders (11) are arranged distributed at fixed angular distances or division distances about the machine axis or rotor axis (MA), and the application stations (23, 24) following one another are spaced apart from one another by at least two angular distances or division distances.

5. Device according to any one of claims 3 or 4, **characterised in that** the individual outlets (21) of the sterilising agent spray device (20, 20') and/or the sterilising agent spray devices (20, 20') of the different application stations (23, 24) are connected to a central sterilising agent feed device.

6. Device according to claim 4, **characterised in that**, in the circulating direction,
following on from the at least one application station (23, 24), and before the at least
one activating station and drying station (27), at least one dwell station (26) is provided.

7. Device according to any one of claims 4 or 5, **characterised in that**, in the circulating direction (A), following the at least one activating and/or drying station (27), at least one cooling station (29) is provided.

8. Device according to anyone of the preceding claims, **characterised in that** the seal holders (11) are oriented with their longitudinal extensions parallel or essentially parallel to the rotor axis (MA).

9. Device according to anyone of the preceding claims, **characterised in that** the sterilising agent spraying devices (20, 20') are provided on the movement path of the seal holders (11) located on the inside and/or the outside, and wherein these do not circulate with these seal holders (11) or with the rotor (10).

10. Device according to anyone of the preceding claims, **characterised by** one or more central gas lines and/or gas line elements, and conveying, controlling, and/or regulating devices connected to them, for gas and atmosphere management in the interior of the device.

11. Method for the sterilisation of seals, **characterised in that** a device is used in accordance with any one of the preceding claims.

## Revendications

1. Dispositif servant à stériliser et/ou désinfecter des systèmes de fermeture (2) servant à fermer des bouteilles ou des contenants similaires, en particulier servant à stériliser des systèmes de fermeture de type capuchon, avec un système de transport servant à déplacer les systèmes de fermeture (2) à travers plusieurs zones de traitement, dans lesquelles les systèmes de fermeture (2) sont au moins stérilisés ainsi qu'activés et séchés, dans lequel le système de transport présente dans l'au moins une zone de traitement au moins un rotor (10) pouvant être entraîné en rotation autour d'un axe de machine ou de rotor (MA) avec une pluralité de logements de système de fermeture (11) réalisés au niveau de la périphérie dudit rotor (10), orientés de manière verticale, au moyen desquels une multitude de systèmes de fermeture (2) peuvent être logés de manière empilée en tant que groupe et peuvent être déplacés dans la direction périphérique (A) en rotation à travers plusieurs zones de traitement sur un trajet de traitement entre un système de distribution de système de fermeture (31) et un système d'évacuation de système de fermeture (33), dans lequel l'au moins un rotor (10) est exécuté à la manière d'un tambour creux ou d'un cylindre creux avec une enveloppe de tambour de type cage formée par une pluralité de logements de système de fermeture (11), dans lequel les logements de système de fermeture (11) sont réalisés quant à eux de manière préférée respectivement à la manière de cages avec des rails de guidage de système de fermeture (14) de type barre et/ou tige s'étendant dans le sens longitudinal du logement de système de fermeture (11) respectif, entre lesquels les systèmes de fermeture (2) sont logés dans le logement de système de fermeture (11) respectif, et dans lequel le dispositif comprend par ailleurs un boîtier ou une enceinte, lesquels présentent une conception polygonale ou cylindrique renfermant l'axe de machine ou de rotor (MA) vertical, dans lequel l'espace intérieur du boîtier à l'exception d'entrées et de sorties pour les systèmes de fermeture (2) est fermé de manière étanche par rapport à l'environnement par une paroi périphérique entourant l'axe de machine (MA) et par une paroi de boîtier supérieure et une paroi de boîtier inférieure, et dans lequel le rotor (10) pouvant être entraîné en rotation autour de l'axe de machine (MA) vertical est logé dans l'espace intérieur du boîtier,
**caractérisé en ce que**
au moins un poste d'application (23, 24) avec un dispositif de pulvérisation de produit de stérilisation (20, 20') dirigé sur au moins un logement de système de fermeture (11) servant à soumettre les systèmes de fermeture (2) individuels logés dans le logement de système de fermeture (11) à l'action d'un produit de stérilisation est prévu au niveau de l'au moins un tronçon de traitement, dans lequel le dispositif de pulvérisation de produit de stérilisation (20, 20') présente respectivement une pluralité de sorties (21), dans lequel les sorties (21) se suivant les unes les autres sont espacées respectivement d'un diamètre de système de fermeture ou sensiblement d'un diamètre de système de fermeture en direction de l'extension longitudinale des logements de système de fermeture (11), et
dans lequel
dans la direction périphérique (A) en suivant l'au moins un poste d'application (23, 24) est prévu au moins un poste d'activation et de séchage (27), au niveau duquel les systèmes de fermeture peuvent être séchés avec un milieu de séchage et d'activation.

2. Dispositif selon la revendication 1, **caractérisé en ce que** sont prévus au niveau de l'au moins un poste d'application (23, 24) au moins deux dispositifs de pulvérisation de produit de stérilisation (20, 20') opposés l'un l'autre, qui sont réalisés pour soumettre les systèmes de fermeture (2) individuels logés dans un logement de système de fermeture (11) à l'action du produit de stérilisation depuis des côtés opposés.

3. Dispositif selon la revendication 1 ou 2, **caractérisé en ce que** plusieurs postes d'application (23, 24) servant à soumettre les systèmes de fermeture (2) logés dans les logements de système de fermeture (11) à l'action du produit de stérilisation sont prévus en se suivant les uns les autres dans la direction périphérique (A) du rotor (10).

4. Dispositif selon la revendication 3, **caractérisé en ce que** les logements de système de fermeture (11) sont disposés de manière répartie à des distances angulaires ou de séparation fixes autour de l'axe de machine ou de rotor (MA) et les postes d'application (23, 24) se suivant les uns les autres sont espacés les uns des autres d'au moins deux distances angulaires ou de séparation.

5. Dispositif selon l'une quelconque des revendications 3 ou 4, **caractérisé en ce que** les sorties (21) individuelles du dispositif de pulvérisation de produit de stérilisation (20, 20') et/ou les dispositifs de pulvérisation de produit de stérilisation (20, 20') de différents postes d'application (23, 24) sont reliés à une amenée de produit de stérilisation centrale.

6. Dispositif selon la revendication 4, **caractérisé en ce qu'**au moins un poste de séjour (26) est prévu dans la direction périphérique en suivant l'au moins un poste d'application (23, 24) avant l'au moins un poste d'activation et de séchage (27).

7. Dispositif selon la revendication 4 ou 5, **caractérisé en ce qu'**au moins un poste de refroidissement (29) est prévu dans la direction périphérique (A) en suivant l'au moins un poste d'activation et/ou de séchage (27).

8. Dispositif selon l'une quelconque des revendications précédentes, **caractérisé en ce que** les logements de système de fermeture (11) sont orientés avec leur extension longitudinale de manière parallèle ou de manière sensiblement parallèle par rapport à l'axe de rotor (MA).

9. Dispositif selon l'une quelconque des revendications précédentes, **caractérisé en ce que** les dispositifs de pulvérisation de produit de stérilisation (20, 20') sont prévus à l'intérieur et/ou à l'extérieur au niveau de la trajectoire de déplacement des logements de système de fermeture (11) et dans lequel ceux-ci ne circulent pas avec lesdits logements de système de fermeture (11) ou avec le rotor (10).

10. Dispositif selon l'une quelconque des revendications précédentes, **caractérisé par** un ou plusieurs conduits de gaz et/ou éléments de conduit de gaz ainsi que par des dispositifs de refoulement, de commande et/ou de régulation reliés à ceux-ci pour la gestion de gaz et d'atmosphère dans l'intérieur du dispositif.

11. Procédé servant à stériliser des systèmes de fermeture, **caractérisé en ce qu'**est utilisé un dispositif selon l'une quelconque des revendications précédentes.
